# EUROPÄISCHE PATENTSCHRIFT

(11) **EP 1 999 112 B1**
(45) Veröffentlichungstag und Bekanntmachung des Hinweises auf die Patenterteilung: **22.04.2015**
(21) Anmeldenummer: 07723091.0
(22) Anmeldetag: 08.03.2007
(51) Int. Cl.: C07D 215/38, A61K 31/47, A61P 1/16, A61P 13/12

(54) **SUBSTITUIERTE 2-AMINO-4-PHENYL-DIHYDROCHINOLINE, VERFAHREN ZU IHRER HERSTELLUNG, IHRE VERWENDUNG ALS MEDIKAMENT, SOWIE SIE ENTHALTENDES MEDIKAMENT**
SUBSTITUTED 2-AMINO-4-PHENYL-DIHYDROQUINOLINES, METHOD FOR THE PRODUCTION THEREOF, USE THEREOF AS MEDICAMENTS, AND MEDICAMENTS CONTAINING THEM
2-AMINO-4-PHÉNYL-DIHYDROQUINOLÉINES SUBSTITUÉES, PROCÉDÉ DE PRÉPARATION, UTILISATION EN TANT QUE MÉDICAMENT ET MÉDICAMENT LES CONTENANT

(30) Priorität: 18.03.2006 DE 102006012545
(43) Veröffentlichungstag der Anmeldung: 10.12.2008
(73) Patentinhaber: SANOFI, 75008 Paris (FR)
(72) Erfinder: LANG, Hans-Jochen, 65926 Frankfurt am Main (DE); WESTON, John, 65926 Frankfurt am Main (DE); HEINELT, Uwe, 65926 Frankfurt am Main (DE)
(74) Vertreter: Michalski Hüttermann & Partner Patentanwälte mbB
(86) Internationale Anmeldenummer: PCT/EP2007/001983
(87) Internationale Veröffentlichungsnummer: WO 2007/107246

(56) Entgegenhaltungen:
- WO-A-03/048129
- US-A- 3 538 101

## Beschreibung

Die Erfindung betrifft Verbindungen vom Typ der substituierten 2-Amino-4-phenyldihydrochinoline. Medikamente, die Verbindungen dieses Typs enthalten, sind nützlich bei der Prävention oder Behandlung diverser Erkrankungen. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I 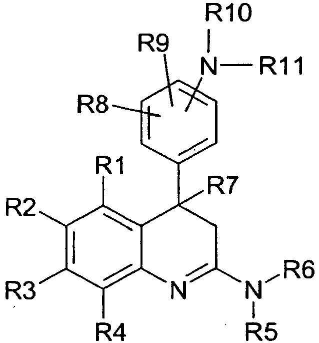 worin bedeuten:
R1, R2, R3 und R4
   unabhängig voneinander Wasserstoff, F, Cl, Br, I, CN, NO₂, CF₃, CH₃-SO₂,
   Alkyl mit 1, 2, 3 oder 4 C-Atomen, NH₂, NH-CH₃ oder N(CH₃)₂; R5 und R6
   unabhängig voneinander Wasserstoff, Alkyl mit 1, 2, 3, 4, 5 oder 6 C-Atomen,
   CF₃-CH₂-, Cycloalkyl mit 3, 4, 5 oder 6 C-Atomen oder Cyclopropyl-CH₂-, oder
R5 und R6
   bilden gemeinsam mit dem Stickstoffatom, an das sie gebunden sind, einen 4-, 5-, 6-, 7-, 8-, 9- oder 10-gliedrigen Ring, in dem ein oder zwei CH₂-Gruppen unabhängig voneinander durch NR12, Schwefel, Sauerstoff, C(O) oder SO₂ ersetzt sein können;
   R12 Wasserstoff, Alkyl mit 1, 2, 3 oder 4 C-Atomen oder Cycloalkyl mit 3, 4, 5 oder 6 C-Atomen;
R7 Wasserstoff oder Alkyl mit 1,2,3 oder 4 C-Atomen;
R8 und R9
   unabhängig voneinander Wasserstoff, F, Cl, Br, OH, Alkyl mit 1, 2, 3 oder 4 C-Atomen, CH₃O, CF₃, oder CH₃SO₂;
R10 und R11
   unabhängig voneinander R13-(CₘH₂ₘ)-Bₙ, wobei
   m Null, 1, 2, 3 oder 4;
   n Null oder 1;
   B -CO-, -CONR14- oder -SO₂-;
      R14 Wasserstoff oder Alkyl mit 1, 2, 3, 4, 5 oder 6 C-Atomen;
   R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl), 1-Morpholinyl -COOR15, OR16, NR17R18 oder Phenyl, das unabhängig voneinander 1 oder 2 Substituenten trägt, ausgewählt aus der Gruppe Chlor, Fluor, Methyl und Methoxy;
      R15, R16, R17 und R18
      unabhängig voneinander Wasserstoff oder Alkyl mit 1, 2, 3, 4, 5 oder 6 C-Atomen;
   oder
R10 und R11
   bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19, Schwefel, Sauerstoff, C(O) oder SO₂ ersetzt sein können;
   R19 Wasserstoff, Alkyl mit 1, 2, 3 oder 4 C-Atomen oder Cycloalkyl mit 3, 4, 5 oder 6 C-Atomen;
sowie deren pharmazeutisch verträgliche Salze und Trifluoracetate.

Bevorzugt sind Verbindungen der Formel I, worin bedeuten
R1, R2, R3 und R4
   unabhängig voneinander Wasserstoff, F, Cl, Br, CN, CF₃, CH₃-SO₂, Alkyl mit 1, 2, 3 oder 4 C-Atomen, NH₂, NH-CH₃ oder N(CH₃)₂;
R5 und R6
   unabhängig voneinander Wasserstoff, Alkyl mit 1, 2, 3, 4, 5 oder 6 C-Atomen,
   CF₃-CH₂-, Cycloalkyl mit 3, 4, 5 oder 6 C-Atomen oder Cyclopropyl-CH₂-, oder
R5 und R6
   bilden gemeinsam mit dem Stickstoffatom, an das sie gebunden sind, einen 4-, 5-, 6-, 7-, 8-, 9- oder 10-gliedrigen Ring;
R7 Wasserstoff;
R8 und R9
   unabhängig voneinander Wasserstoff, F, Cl, OH, Alkyl mit 1, 2, 3 oder 4 C-Atomen, CH₃O, CF₃, oder CH₃SO₂;
R10 und 11
   unabhängig voneinander R13-(CₘH₂ₘ)-Bₙ, wobei
   m Null, 1, 2, 3 oder 4;
   n Null oder 1;
   B -CO-, -CONR14- oder -SO₂-;
      R14 Wasserstoff oder Alkyl mit 1, 2, 3, 4, 5 oder 6 C-Atomen;
   R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl), 1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy;
      R15, R16, R17 und R18
      unabhängig voneinander Wasserstoff oder Alkyl mit 1, 2, 3, 4, 5 oder 6 C-Atomen;
   oder
R10 und R11
   bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch Sauerstoff oder NR19 ersetzt sein kann;
   R19 Wasserstoff oder Alkyl mit 1, 2, 3 oder 4 C-Atomen;
sowie deren pharmazeutisch verträgliche Salze und Trifluoracetate.

Besonders bevorzugt sind Verbindungen der Formel I, worin bedeuten
R1, R2, R3 und R4
   unabhängig voneinander Wasserstoff, F, Cl, Br, CN, CF₃, CH₃-SO₂, Methyl, Ethyl, NH₂, NH-CH₃ oder N(CH₃)₂;
R5 und R6
   unabhängig voneinander Wasserstoff, Methyl, Ethyl, Isopropyl, CF₃-CH₂ oder Cycloalkyl mit 3, 4, 5 oder 6 C-Atomen;
R7 Wasserstoff;
R8 und R9
   unabhängig voneinander Wasserstoff, Cl oder Methyl;
R10 und 11
   unabhängig voneinander R13-(CₘH₂ₘ)-Bₙ, wobei
   m Null, 1, 2, 3 oder 4;
   n Null oder 1;
   B -CO-, -CONR14- oder -SO₂-;
      R14 Wasserstoff oder Alkyl mit 1, 2, 3, 4, 5 oder 6 C-Atomen;
   R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16oder NR17R18;
      R15, R16, R17 und R18
      unabhängig voneinander Wasserstoff oder Alkyl mit 1, 2, 3, 4, 5 oder 6 C-Atomen;
   oder
R10 und R11
   bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch Sauerstoff oder NR19 ersetzt sein kann;
   R19 Wasserstoff oder Methyl;
sowie deren pharmazeutisch verträgliche Salze und Trifluoracetate.

Insbesondere bevorzugt sind Verbindungen der Formel I, worin bedeuten
R1 und R3
   Wasserstoff;
R2 und R4
   unabhängig voneinander Wasserstoff oder Cl;
R5 und R6
   unabhängig voneinander Wasserstoff, Methyl oder Ethyl;
R7 Wasserstoff;
R8 und R9
   unabhängig voneinander Wasserstoff oder Cl;
R10 und 11
   unabhängig voneinander R13-(CₘH₂ₘ)-Bₙ, wobei
   m Null, 1, 2, 3 oder 4;
   n Null oder 1;
   B -CONR14-;
      R14 Wasserstoff oder Methyl;
   R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
      R15, R16, R17 und R18
      unabhängig voneinander Wasserstoff oder Alkyl mit 1, 2, 3, 4, 5 oder 6 C-Atomen;
   oder
R10 und R11
   bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch NR19 ersetzt sein kann;
   R19 Wasserstoff oder Methyl;
sowie deren pharmazeutisch verträgliche Salze und Trifluoracetate.

Sehr bevorzugt sind Verbindungen der Formel I, worin bedeuten
R1 und R3
   Wasserstoff;
R2 und R4
   unabhängig voneinander Wasserstoff oder Cl;
R5 und R6
   unabhängig voneinander Wasserstoff, Methyl oder Ethyl;
R7 Wasserstoff;
R8 und R9
   unabhängig voneinander Wasserstoff oder Cl;
R10 und 11
   unabhängig voneinander R13-(CₘH₂ₘ)-Bₙ, wobei
   m Null, 1, 2, 3 oder 4;
   n Null oder 1;
   B -CONR14-;
      R14 Wasserstoff oder Methyl;
   R13 Wasserstoff, Methyl, oder NR17R18;
      R17 und R18
unabhängig voneinander Wasserstoff, Methyl oder Ethyl;
sowie deren pharmazeutisch verträgliche Salze und Trifluoracetate.

Speziell bevorzugt sind Verbindungen der Formel I ausgewählt aus der Gruppe:
2-Amino-4-(4-aminophenyl)-6-chlor-3,4-dihydrochinolin,
4-(4-Aminophenyl)-6-chlor-2-ethylamino-3,4-dihydrochinolin,
4-(4-Aminophenyl)-6-chlor-2-diethylamino-3,4-dihydrochinolin und
N-(2-Dimethylaminoethyl)-N'-4-[(6-chloro-3,4-dihydro-2-diethylaminoquinolin-4-yl)phenyl]harnstoff,
sowie dessen pharmazeutisch verträgliche Salze und Trifluoracetate.

In einer Ausführungsform sind Verbindungen der Formel I bevorzugt, in denen die Reste R1, R2, R3 und R4 unabhängig voneinander beschrieben werden durch Wasserstoff, F, Cl, Br, CN, CF₃, CH₃-SO₂, Alkyl mit 1, 2, 3 oder 4 C-Atomen, beispielsweise Methyl oder Ethyl, NH₂, NH-CH₃ oder N(CH₃)₂; besonders bevorzugt sind Verbindungen der Formel I, in denen R1 und R3 Wasserstoff sind und R2 und R4 unabhängig voneinander Wasserstoff, F, Cl, Br, CN, CF₃, CH₃-SO₂, Methyl, Ethyl, NH₂, NH-CH₃ oder N(CH₃)₂, beispielsweise Wasserstoff oder Cl, sind; in einer weiteren Ausführungsform sind Verbindungen der Formel I bevorzugt, in denen R1, R3 und R4 Wasserstoff sind und R2 F, Cl, Br, CN, CF₃, CH₃-SO₂, Methyl, Ethyl, NH₂, NH-CH₃ oder N(CH₃)₂, beispielsweise Cl, ist.

In einer Ausführungsform sind Verbindungen der Formel I bevorzugt, in denen R5 und R6 unabhängig voneinander durch Wasserstoff, Alkyl mit 1, 2, 3, 4, 5 oder 6 C-Atomen, CF₃-CH₂- oder Cycloalkyl mit 3, 4, 5 oder 6 C-Atomen beschrieben werden oder R5 und R6 gemeinsam mit dem Stickstoffatom, an das sie gebunden sind, einen 4-, 5-, 6-, 7-, 8-, 9- oder 10-gliedrigen Ring bilden; in einer weiteren Ausführungsform sind Verbindungen der Formel I bevorzugt, in denen R5 und R6 unabhängig voneinander Wasserstoff, Methyl, Ethyl, Isopropyl, CF₃-CH₂ oder Cycloalkyl mit 3, 4, 5 oder 6 C-Atomen sind, insbesondere Wasserstoff, Methyl oder Ethyl, beispielsweise Wasserstoff oder Ethyl.

In einer weiteren Ausführungsform sind Verbindungen der Formel I bevorzugt, in denen R7 durch Wasserstoff oder Methyl, beispielsweise Wasserstoff, beschrieben wird.

In einer Ausführungsform sind Verbindungen der Formel I bevorzugt, in denen die Reste R8 und R9 unabhängig voneinander beschrieben werden durch Wasserstoff, F, Cl, OH, Alkyl mit 1, 2, 3 oder 4 C-Atomen, CH₃O, CF₃, oder CH₃SO₂; besonders bevorzugt sind Verbindungen der Formel I, in denen R8 und R9 unabhängig voneinander beschrieben werden durch Wasserstoff, Cl oder Methyl, insbesondere Wasserstoff oder Cl, beispielsweise Wasserstoff.

Der Rest NR10R11 am Phenylring kann in ortho-, meta- oder para-Position zur Dihydroisochinolingruppe gebunden sein, beispielsweise in ortho- oder para-Position, insbesondere in para-Position.

In einer Ausführungsform sind Verbindungen der Formel I bevorzugt, in denen die Reste R10 und R11 unabhängig voneinander beschrieben werden durch R13-(CₘH₂ₘ)-Bₙ oder R10 und R11 bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch Sauerstoff oder NR19 ersetzt sein kann, wobei R19 Wasserstoff oder Alkyl mit 1, 2, 3 oder 4 C-Atomen, insbesondere Wasserstoff oder Methyl, ist; in einer weiteren Ausführungsform sind Verbindungen der Formel I bevorzugt, in denen die Reste R10 und R11 unabhängig voneinander beschrieben werden durch R13-(CₘH₂ₘ)-Bₙ.

In einer Ausführungsform sind Verbindungen der Formel I bevorzugt, in denen m Null, 1 oder 2 ist, beispielsweise Null oder 2.

In einer Ausführungsform sind Verbindungen der Formel I bevorzugt, in denen n Null ist; in einer weiteren Ausführungsform sind Verbindungen der Formel I bevorzugt, in denen n 1 ist.

In einer Ausführungsform sind Verbindungen der Formel I bevorzugt, in denen B -CONR14- ist, wobei R14 Wasserstoff oder Alkyl mit 1, 2, 3, 4, 5 oder 6 C-Atomen, insbesondere Wasserstoff oder Methyl, beispielsweise Wasserstoff, ist.

in einer Ausführungsform sind Verbindungen der Formel i bevorzugt, in denen R13 beschrieben wird durch Wasserstoff, Methyl, Ethyl, Isopropyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18, wobei R15, R16, R17 und R18 unabhängig voneinander Wasserstoff oder Alkyl mit 1, 2, 3, 4, 5 oder 6 C-Atomen sind; in einer weiteren Ausführungsform sind Verbindungen der Formel I bevorzugt, in denen R13 beschrieben wird durch Wasserstoff, Methyl oder NR17R18, wobei R17 und R18 unabhängig voneinander Wasserstoff oder Alkyl mit 1, 2, 3, 4, 5 oder 6 C-Atomen, insbesondere Wasserstoff, Methyl oder Ethyl, beispielsweise Methyl, sind; besonders bevorzugt sind Verbindungen der Formel I, in denen R13 Wasserstoff oder Dimethylamino ist.

Enthalten die Verbindungen der Formel I ein oder mehrere Asymmetriezentren, so können diese unabhängig voneinander sowohl S als auch R konfiguriert sein. Die Verbindungen können als optische Isomere, als Diastereomere, als Racemate oder als Gemische in allen Verhältnissen derselben vorliegen. Die Verbindungen der Formel I können weiterhin in Form von Rotationsisomeren vorliegen.

Die vorliegende Erfindung umfasst alle möglichen tautomeren Formen der Verbindungen der Formeln I.

Die vorliegende Erfindung umfasst weiterhin Derivate der Verbindungen der Formel I, zum Beispiel Solvate, wie Hydrate und Alkoholaddukte der Verbindungen der Formel I. Die Erfindung umfasst ebenfalls alle Kristallmodifikationen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl oder Hexyl. Bevorzugte Alkylreste sind Methyl, Ethyl, n-Propyl, Isopropyl, n-Butyl, tert-Butyl, n-Pentyl, n-Hexyl.

Beispiele für Cycloalkylreste sind Cyclopropyl, Cyclobutyl, Cyclopentyl, Cyclohexyl, Cycloheptyl oder Cyclooctyl. In den Cycloalkylresten können eine oder mehrere CH₂-Gruppen durch O, NH oder N-Alkyl, beispielsweise NCH₃, ersetzt sein. Dies gilt auch für Cycloalkylmethylreste.

Beispiele für Ringe aus NR5R6 sind Morpholin, Pyrrolidin, Piperidin, Piperazin, N-Methylpiperazin, Pyrrolidin-2-on, Pyrrolidin-2,5-dion, Imidazolidin, 3-Methylimidazolidin, Imidazolidin-2-on, 3-Methyl-Imidazolidin-2-on, Imidazolidin-2,4-dion und 1-Methyl-imidazolidine-2,4-dion, insbesondere Pyrrolidin und Piperidin, beispielsweise Pyrrolidin.

Beispiele für Ringe aus NR10R11 sind Morpholin, Pyrrolidin, Piperidin, Piperazin, N-Methylpiperazin, Pyrrolidin-2-on, Pyrrolidin-2,5-dion, Imidazolidin, 3-Methylimidazolidin, Imidazolidin-2-on, 3-Methyl-Imidazolidin-2-on, Imidazolidin-2,4-dion und 1-Methyl-imidazolidine-2,4-dion, insbesondere Pyrrolidin-2,5-dion und Imidazolidin-2,4-dion, beispielsweise Imidazolidin-2,4-dion.

Als CH₂-Einheiten gelten auch die in einem Alkylrest terminalen CH₃-Gruppen, die in diesem Zusammenhang als CH₂-H Gruppierungen aufgefasst werden.

Wenn eine Variable, beispielsweise cycloalkyl oder R1, mehr als einmal als Komponente vorkommt, sind die Definitionen der Variable bei jedem Auftreten unabhängig voneinander.

Enthalten die Verbindungen der Formel I eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. So können die Verbindungen der Formel I an einer sauren Gruppe deprotoniert werden und beispielsweise als Alkalimetallsalze, vorzugsweise Natrium- oder Kaliumsalze, oder als Ammoniumsalze, zum Beispiel als Salze mit Ammoniak oder organischen Aminen oder Aminosäuren, verwendet werden. Da Verbindungen der Formel I stets wenigstens eine basische Gruppe enthalten, können sie auch in Form ihrer physiologisch verträglichen Säureadditionssalze z. B. mit folgenden Säuren hergestellt werden: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage (diese Gruppe entspricht auch den physiologisch akzeptablen Anionen), beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate; aber auch Trifluoracetate.

Gegenstand der Erfindung ist auch das nachfolgend beschriebene Verfahren zur Herstellung der Verbindungen der Formel I.

Die hier beschriebenen Verbindungen der Formel I, in denen R10 und R11 Wasserstoff sind, lassen sich beispielsweise ausgehend von den Chinolin-Derivaten der Formel II durch Reduktion zu den entsprechenden 3,4-Dihydrochinolinen der Formel la herstellen, 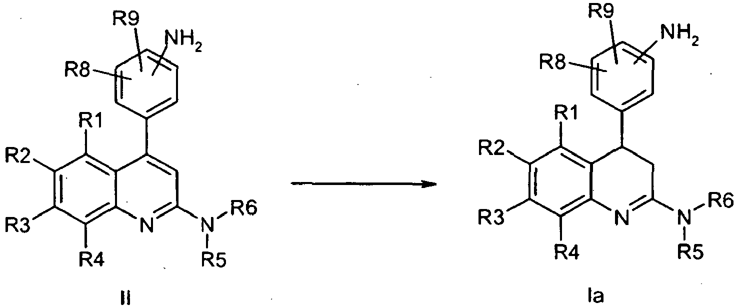 wobei die Substituenten R1, R2, R3, R4, R5, R6, R8 und R9 wie oben beschrieben definiert sind. Die Reduktion von Chinolin-Derivate der Formel II zu den entsprechenden 3,4-Dihydrochinolinen der Formel la kann beispielsweise mit einer Natrium-Amalgam-Legierung durchführt werden (US3,538,101).

Aus den erfindungsgemäßen Verbindungen der Formel I a) fassen sich weitere erfindungsgemäßen Verbindungen der Formel I beispielweise durch Derivatisierung der Aminogruppe am Phenylrest nach dem Fachmann bekannten Verfahren darstellen. Dabei wird beispielsweise die Aminogruppe der Verbindungen Ia) mit Alkylierungsmitteln, Acylierungsmitteln bzw. Sulfonylierungs-Reagentien der Formel R10-L und/oder R11-L vorteilhaft in Gegenwart einer Hilfsbase, wie Pyridin, Triethylamin oder Hünigscher Base, in dem Fachmann bekannter Weise umgesetzt. Ebenfalls eignet sich die Verwendung von Isocyanaten der Formeln R10-N=C=O und/oder R11-N=C=O in dem Fachmann bekannter Weise zur Herstellung entsprechender Harnstoffderivate der Formel Ib oder Ic. 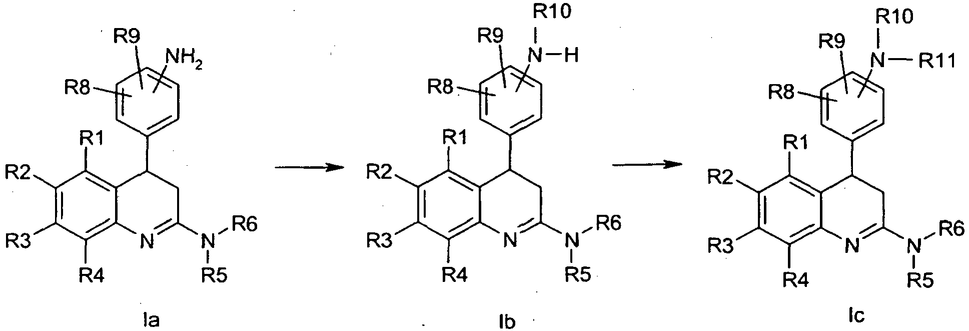 wobei die Substituenten R1, R2, R3, R4, R5, R6, R8, R9, R10 und R11 die oben angegebene Bedeutung haben, R10 und R11 jedoch nicht Wasserstoff sind, und L F, Cl, Br, I, -OR, -OC(O)R oder -SR bedeutet, wobei R Alkyl mit 1, 2, 3, 4, 5 oder 6 C-Atomen, beispielsweise Methyl oder Ethyl, bedeutet und L dann -SR bedeuten kann, wenn B -C(O)- ist. Durch stufenweise erfolgende Umsetzungen kann beispielweise die monosubstituierte erfindungsgemäße Verbindung der Formel Ib erhalten und isoliert werden und/oder anschließend zur disubstituierten Verbindung der Formel Ic umgesetzt werden.

Analog können die Verbindungen der Formel II durch Derivatisierung der Aminogruppe am Phenylrest nach dem Fachmann bekannten Verfahren zu Verbindungen der Formel IIIa oder IIIb umsetzt werden, 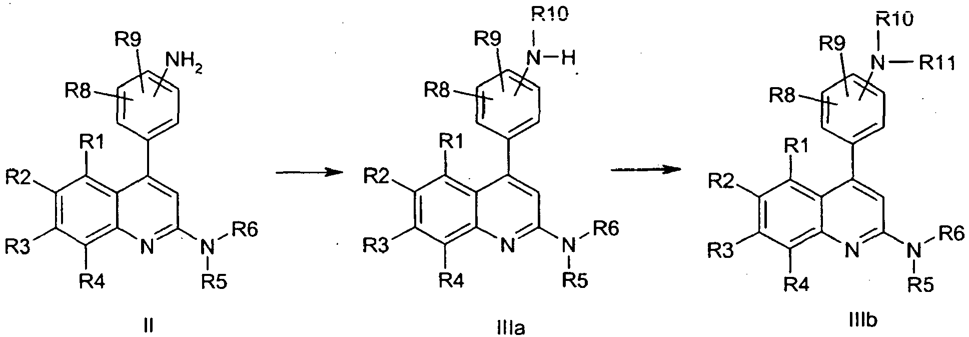 wobei die Substituenten R1, R2, R3, R4, R5, R6, R8, R9, R10 und R11 die oben angegebene Bedeutung haben, R10 und R11 jedoch nicht Wasserstoff sind.

Die auf diese Weise erhaltenen Chinolin-Derivate der Formel IIIa oder IIIb, in denen R10 und R11 nicht in der Bedeutung von Wasserstoff haben, können anschließend in bekannter Weise, beispielsweise mit Natrium-Amalgam-Legierung, zu den erfindungsgemäßen entsprechenden 3,4-Dihydrochinolin-Derivaten der Formel Ib oder Ic reduziert werden, 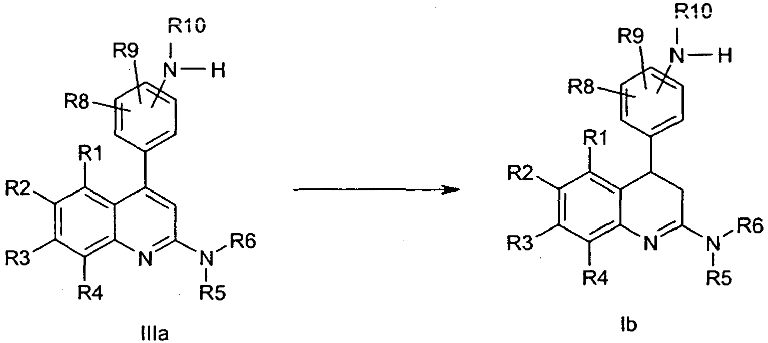 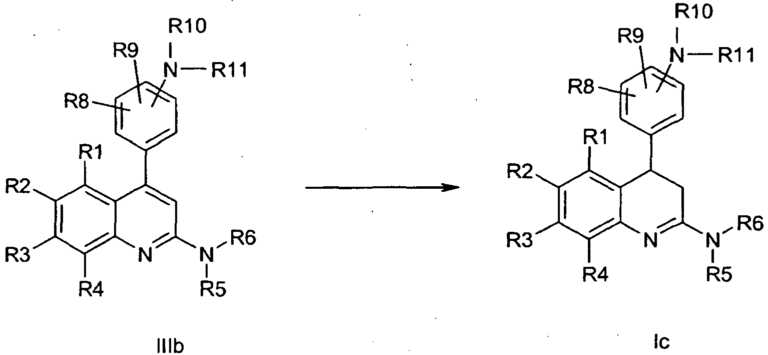 wobei die Substituenten R1, R2, R3, R4, R5, R6, R8, R9, R10 und R11 die oben angegebene Bedeutung haben, R10 und R11 jedoch nicht Wasserstoff sind.

Die oben verwendeten Aniline der Formel II werden vorzugsweise durch Reduktion der entsprechenden Nitroverbindungen der Formel IVa gewonnen, 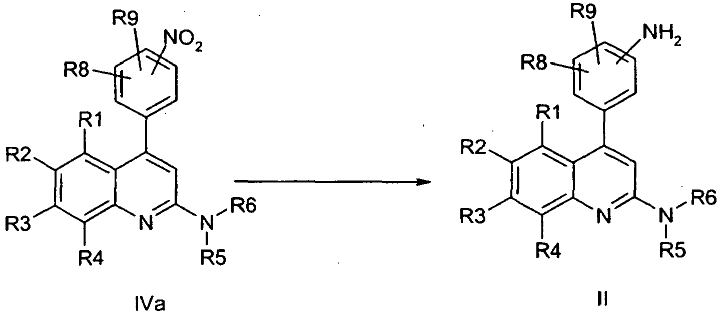 wobei die Substituenten R1, R2, R3, R4, R5, R6, R8, R9 die angegebene Bedeutung besitzen. Die Reduktion kann nach dem Fachmann bekannten Verfahren erfolgen, beispielsweise durch katalytische Hydrierung oder mit einem anorganischen Reduktionsmittel, wie zum Beispiel mit Eisenpulver und Salzsäure in Eisessig.

Zur Herstellung von Verbindungen der Formel IVa, in denen R5 und R6 Wasserstoff bedeuten, kann man beispielsweise Verbindungen der Formel V in einer alkalischen Kondensation mit Acetonitril (I.A.Nicolls et al., Life Sciences 53, 343-347 (1993)) zu Verbindungen der Formel IVb umsetzen, 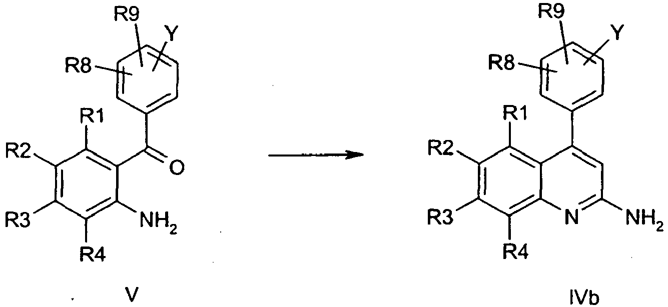 wobei R1, R2, R3, R4, R8 und R9 die oben beschriebene Bedeutung haben und Y Wasserstoff oder NO₂ bedeutet.

Die Verbindungen der Formel IVb, in denen Y Wasserstoff bedeutet, können durch Nitrierung zu Nitro-substituierten Verbindungen der Formel IVa, in denen R5 und R6 Wasserstoff bedeuten, umgesetzt werden.

Die Verbindungen der Formel IVa, in denen R5 und R6 nicht beide Wasserstoff bedeuten, erhält man beispielsweise durch nucleophile Austauschreaktionen von Chinolin-Derivaten der Formel VI mit einem Amin der Formel VII 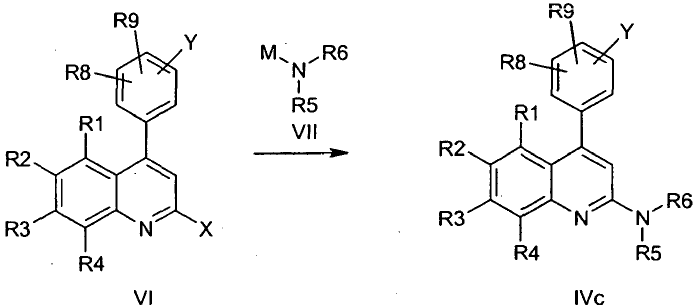 wobei R1, R2, R3, R4, R5, R6, R8, R9 und Y die oben angegebene Bedeutung besitzen, R5 und R6 jedoch nicht beide Wasserstoff bedeuten, X eine nucleophil substituierbare Fluchtgruppe repräsentiert, wie beispielsweise Chlorid, Bromid, Tosylat, Mesylat, Triflat, Alkoxy mit 1, 2, 3, 4, 5 oder 6 C-Atomen, zum Beispiel Ethoxy, Aryloxy, zum Beispiel Phenoxy, oder RS(O)ₙ-, wobei n 0 oder 2 bedeutet und R' ein Alkylrest, vorzugsweise mit 1, 2, 3 oder 4 C-Atomen, beispielsweise Methyl, ist, und M entweder Wasserstoff oder ein Metall, insbesondere ein Alkalimetall oder ein Erdalkalimetalläquivalent bedeutet, beispielsweise Lithium, oder die Verbindung VII eine Grignardverbindung ist. Vorteilhaft arbeitet man mit Aminen unter Anwendung von Temperaturen >100°C, wobei die Verwendung eines Autoklaven oder einer Microwelle emfohlen werden kann.

Entsprechende Verbindungen der Formel IVb, in denen R5 und R6 Wasserstoff bedeuten, können analog durch Umsetzung mit NH₃ und unter Druck hergestellt werden.

Die Verbindungen der Formel IVc, in denen Y Wasserstoff bedeutet, können durch Nitrierung zu Nitro-substituierten Verbindungen der Formel IVa, in denen R5 und R6 nicht beide Wasserstoff bedeuten, umgesetzt werden.

Verbindungen VI kann der Fachmann bekannter Weise hergestellt werden, zum Beispiel aus Verbindungen der Formel VIII beispielsweise durch Einwirkung eines anorganischen Säurechlorides, wie Phosphoroxid-trichlorid, Phosphortrichlorid oder Phosphorpentachlorid' für Verbindungen der Formel VI, in denen X Cl ist, 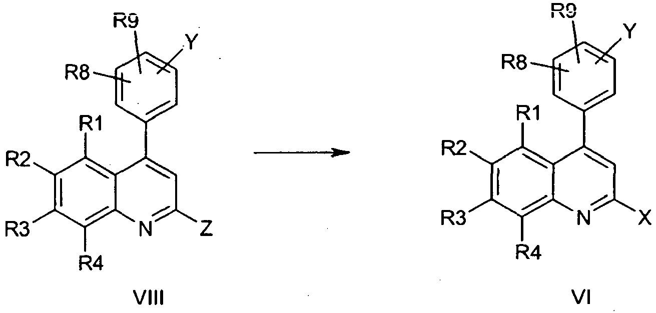 wobei R1, R2, R3, R4, R8, R9, Y und X die angegebene Bedeutung besitzen und Z eine OH-Gruppe bedeutet.

Verbindungen der Formel I, in denen R7 nicht Wasserstoff ist, können ausgehend von dem Fachmann bekannten Verbindungen, zum Beispiel Verbindungen wie in Helv. Chim. Act. 1970, 53, 89, nach bekannten Verfahren hergestellt werden.

Die Verbindungen R10-Hal, R11-Hal, R10-N=C=O, R11-N=C=O und die Verbindungen der Formeln V, VII und VIII sind käuflich erhältlich oder können nach oder analog zu in der Literatur beschriebenen, dem Fachmann bekannten Verfahren hergestellt werden.

Die Aufarbeitung und gewünschtenfalls die Reinigung der Produkte und/oder Zwischenprodukte erfolgt nach den üblichen Methoden wie Extraktion, Chromatographie oder Kristallisation und den üblichen Trocknungen.

Es konnte gezeigt werden, dass Verbindungen der Formel I hervorragende Inhibitoren des Natrium-Wasserstoffaustauschers (NHE), insbesondere des Natrium-Wasserstoffaustauschers vom Subtyp 3 (NHE3), darstellen.

Die bisher bekannten NHE3-Inhibitoren leiten sich beispielsweise von Verbindungen des Acylguanidin-Typs (EP825178), Norbornylamin-Typs (WO0144164), 2-Guanidino-chinazolin-Typs (WO0179186) oder Benzamidin-Typs (WO0121582, WO0172742) ab. Das ebenfalls als NHE3-Inhibitor beschriebene Squalamin (M. Donowitz et al. Am. J. Physiol. 276 (Cell Physiol. 45): C136 - C144) wirkt nach derzeitigem Wissensstand nicht unmittelbar wie die Verbindungen nach Formel I, sondern über einen indirekten Mechanismus und erreicht so seine maximale Wirkstärke erst nach einer Stunde.

Tetrahydroisochinoline als Inhibitoren des Natrium-Wasserstoffaustauschers vom Subtyp 3 (NHE3) werden beispielsweise in den Patentanmeldungen WO03048129, WO2004085404 und den deutschen Anmeldungen 102004046492.8 und 102005001411.9 beschrieben. In der Patentanmeldung WO03055880 ist die verwandte Verbindungsklasse der Tetrahydroisochinolinium-Salze als NHE3-Inhibitoren beschrieben.

Überraschend wurde nun gefunden, dass die hier beschriebenen Verbindungen der Formel I ebenfalls potente Inhibitoren des NHE3 darstellen und dabei vorteilhafte pharmakologische und pharmakokinetische Eigenschaften besitzen.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund ihrer NHE-inhibitorischen Eigenschaften eignen sich die Verbindungen der Formel I zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.
Die Verbindungen der Formel I können auch zur Behandlung und Prävention von Krankheiten eingesetzt werden, wobei der NHE nur partiell gehemmt wird, beispielsweise durch Verwendung einer niedrigeren Dosierung.

Die Verwendung der erfindungsgemäßen Verbindungen betrifft die Prävention und die Behandlung akuter und chronischer Krankheiten in der Veterinär- und in der Humanmedizin.

Infolge Ihrer pharmakologische Wirkungen sind die Verbindungen der Formel I insbesondere dazu geeignet, zu einer Verbesserung des Atemantriebes führen. Sie können deshalb zur Behandlung von gestörten Atmungszuständen herangezogen werden, wie sie beispielsweise bei folgenden klinischen Zuständen und Krankheiten auftreten können: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird. Die genannten Verbindungen finden deshalb vorteilhaft Verwendung zur Herstellung eines Medikaments zur Prävention und Behandlung von Schlafapnoen und muskulär bedingter Atemstörungen und zur Herstellung eines Medikaments zur Prävention und Behandlung des Schnarchens.

Eine Kombination eines NHE-Inhibitors der Formel I mit einem Carboanhydrase-Hemmer (z. B. Acetazolamid) kann sich als vorteilhaft erweisen, wobei letzterer eine metabolische Azidose herbeiführt und dadurch bereits die Atmungstätigkeit steigert, so dass eine verstärkte Wirkung und verminderter Wirkstoffeinsatz erzielt werden können.

Die erfindungsgemäßen Verbindungen schonen infolge ihrer NHE3-inhibitorischen Wirkung die zellulären Energiereserven, die sich unter toxischen und pathogenen Ereignissen rasch erschöpfen und so zur Zellschädigung oder zum Zelluntergang führen. Dabei wird die energieaufwendige ATP-verbrauchende Natriumresorption im proximalen Tubulus unter dem Einfluß von NHE3-Inhibitoren vorübergehend still gelegt und die Zelle kann so eine akute pathogene, ischämische oder toxische Situation überleben. Die Verbindungen eignen sich deshalb beispielsweise als Arzneimittel zur Behandlung ischämischer Noxen, zum Beispiel des akuten Nierenversagens. Weiterhin eignen sich die Verbindungen auch zur Behandlung aller chronischen Nierenerkrankungen und Nephritisformen, die infolge vermehrter Proteinausscheidung zum chronischen Nierenversagen führen. Dementsprechend eignen sich die Verbindungen der Formel I zur Herstellung eines Medikaments zur Behandlung diabetischer Spätschäden, der diabetischen Nephropathie und chronischer Nierenerkrankungen, insbesondere von allen Nierenentzündungen (Nephritiden), die mit einer vermehrten Protein-/ Albuminausscheidung verbunden sind.

Es hat sich gezeigt, dass die erfindungsgemäß verwendeten Verbindungen eine milde abführende Wirkung besitzen und demzufolge auch vorteilhaft als Abführmittel oder bei drohender Darmverstopfung verwendet werden können.

Weiterhin können die erfindungsgemäßen Verbindungen vorteilhaft zur Prävention und Therapie von akuten und chronischen Erkrankungen des Darmtrakts verwendet werden, die beispielweise durch ischämische Zustände im Intestinalbereich und / oder durch nachfolgende Reperfusion oder durch entzündliche Zustände und Ereignisse ausgelöst werden. Solche Komplikationen können beispielweise durch mangelnde Darmperistaltik auftreten, wie sie z.B. häufig nach chirurgischen Eingriffen, bei Darmverstopfung oder stark verminderter Darmtätigkeit zu beobachten sind.

Mit den erfindungsgemäßen Verbindungen besteht die Möglichkeit, der Gallenstein-Bildung vorzubeugen.

Die erfindungsgemäßen NHE-Inhibitoren eignen sich generell zur Behandlung von Krankheiten, die durch Ischämie und durch Reperfusion hervorgerufen werden.

Die erfindungsgemäßen Verbindungen sind infolge ihrer pharmakologischen Eigenschaften als antiarrhythmische Arzneimittel geeignet.
Durch ihre cardioprotektive Komponente eignen sich die NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die erfindungsgemäßen Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den mit Verbindungen der Formel I vorbehandelten Empfängerorganismus verwendet werden können.

Die Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Da NHE-Inhibitoren menschliches Gewebe und Organe nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist deren kombinierte Verabreichung mit Verbindungen der Formel I geeignet, die cytotoxischen Effekte einer Therapie zu vermindern oder zu unterdrücken. Durch die Verminderung der cytotoxischen Effekte, insbesondere der Cardiotoxizität, infolge Ko-Medikation mit NHE-Inhibitoren kann außerdem die Dosis der cytotoxischen Therapeutika erhöht und / oder die Medikation mit solchen Arzneimitteln verlängert werden. Der therapeutische Nutzen einer solchen cytotoxischen Therapie kann durch die Kombination mit NHE-Inhibitoren erheblich gesteigert werden.
Die Verbindungen der Formel I eignen sich insbesondere zur Verbesserung der Therapie mit Arzneimitteln, die eine unerwünschte cardiotoxische Komponente besitzen.

Generell können die hier beschriebenen NHE Inhibitoren in günstiger Weise mit anderen, den intrazellulären pH-Wert ebenfalls regulierenden Verbindungen kombiniert werden, wobei Inhibitoren der Enzymgruppe der Carboanhydratasen, Inhibitoren der Bicarbonationen transportierenden Systeme wie des Natrium-Bicarbonat-Cotransporters (NBC) oder des Natrium-abhängigen Chlorid-Bicarbonat-Austauschers (NCBE), sowie mit anderen NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems geeignet sind.

Die Verbindungen der Formel I eignen sich auch zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelöster klonischer und tonischer Spasmen, psychischer Depressionszustände, Angsterkrankungen und Psychosen. Dabei können die erfindungsgemäßen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich die erfindungsgemäßen Verbindungen der Formel I ebenfalls zur Behandlung von Formen des Schocks, wie beispielweise des allergischen, cardiogenen, hypovolämischen und des bakteriellen Schocks.

Die Verbindungen der Formel I können ebenfalls zur Prävention und zur Behandlung thrombotischer Erkrankungen verwendet werden, da sie als NHE-Inhibitoren sowohl die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und thrombogener Selectin-Proteine, hemmen bzw. verhindern. Damit kann die pathogene Wirkung thrombogener und entzündungsrelevanter Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan-Rezeptor-Antagonisten, Phosphodiesterase-Inhibitoren, Faktor-VIIa-Antagonisten, Clopidogrel, Ticlopidin usw. Eine kombinierte Anwendung der vorliegenden NHE-Inhibitoren mit NCBE-Inhibitoren und/oder mit Inhibitoren der Carboanhydratase, wie beispielsweise mit Acetazolamid, ist besonders günstig.

Darüber hinaus zeichnen sich die erfindungsgemäßen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen das chronische Nierenversagen, Krebserkrankungen, verwendet werden. Sie können somit zur Behandlung von Organhypertrophien und -hyperplasien, beispielsweise des Herzens und der Prostata verwendet werden. Verbindungen der Formel I sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen.

Da der NHE bei essentiellen Hypertonikern signifikant erhöht ist, eignen sich die Verbindungen der Formel I zur Prävention und zur Behandlung des Bluthochdrucks und von Herz-Kreislauferkrankungen. Dabei können sie allein oder mit einem geeigneten Kombinationspartner zur Bluthochdruckbehandlung und zur Behandlung von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, mit Verbindungen der Formel I kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch ß-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromokalim, Minoxidil und deren Derivate, Aktivatoren des mitochondrialen ATP-sensitiven Kaliumkanals (mitoK(ATP) Kanal), Inhibitoren weiterer Kaliumkanäle, wie des Kv1.5 usw.

Infolge ihrer antiphlogistischen Wirkung können erfindungsgemäße NHE-Inhibitoren als Antiinflammatorika verwendet werden. Mechanistisch fällt dabei die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet.

Es wurde außerdem gefunden, dass NHE-Inhibitoren eine günstige Beeinflussung der Serumlipoproteine zeigen. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie z.B. beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades. NHE-Inhibitoren der Formel I finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und zur Behandlung kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z.B. Lovastatin oder Pravastatin), wobei letzterer eine hypolipidämische Wirkung herbeiführt und dadurch die hypolipidämischen Eigenschaften des NHE-Inhibitors der Formel I steigert, stellt eine günstige Kombination mit verstärkter Wirkung und vermindertem Wirkstoffeinsatz dar.

So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind NHE-Inhibitoren wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem eignen sich NHE-Inhibitoren zur Behandlung des nicht-insulinabhängigen Diabetes (NIDDM), wobei beispielweise die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR-Agonisten, wie Rosiglitazone, Pioglitazone etc, mit einem Insulinpräparat unterschiedlicher Verabreichungsform, mit einem DB4 Inhibitor, mit einem Insulinsensitizer oder mit Meglitinide zu kombinieren.

Neben den akuten antidiabetischen Effekten wirken NHE-Inhibitoren der Entstehung diabetischer Spätkomplikation entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den oben unter NIDDM-Behandlung beschriebenen antidiabetischen Arzneimitteln vorteilhaft kombiniert werden. Der Kombination mit einer günstigen Darreichungsform von Insulin kann dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die auch unabhängig von akuten Mangeldurchblutungszuständen sind und auch bei normalen, nicht-ischämischen Bedingungen auftreten können. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Relaxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden. Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens. Außerdem eignen sich NHE-Inhibitoren somit hervorragend zur Behandlung und Prävention der Herzinsuffizienz, des congestive heart failure (CHF) sowie zur Behandlung und insbesondere zur Prävention von altersbedingten Formen von Krebs.
Dabei kommt eine Kombination mit blutdrucksenkenden Medikamenten, wie mit ACE-Hemmern, Angiotensinrezeptorantagonisten, Diuretika, Ca²⁺-Antagonisten etc. oder mit stoffwechselnormalisierenden Medikamenten, wie Cholesterinsenkern, in Betracht. Die Verbindungen der Formel I eignen sich somit zur Prävention altersbedingter Gewebsveränderungen und zur Gesunderhaltung und Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Weiterhin sind NHE-Inhibitoren zur Behandlung von durch Bakterien sowie durch Protozoen ausgelösten Erkrankungen (human und veterinär) geeignet. Bei den durch Protozoen ausgelösten Erkrankungen sind insbesondere Malariaerkrankungen des Menschen und Hühnercoccidiose zu nennen.
Außerdem eignen sich die Verbindungen als Mittel zur Bekämpfung von saugenden Parasiten in der Human- und Veterinärmedizin sowie im Pflanzenschutz. Dabei ist die Verwendung als Mittel gegen blutsaugende Parasiten in der Human- und Veterinärmedizin bevorzugt.

Die genannten Verbindungen finden daher vorteilhaft Verwendung allein oder in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämieoder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

Die Erfindung betrifft weiterhin die Verwendung der Verbindungen der Formel I und deren pharmazeutisch verträgliche Salze zur Verwendung als Medikament.

Die Erfindung betrifft auch Heilmittel/pharmazeutische Zubereitungen für die humane, veterinäre oder phytoprotektive Anwendung enthaltend eine wirksame Menge einer Verbindung der Formel I und / oder eines pharmazeutisch verträglichen Salzes davon, ebenso wie Pharmazeutische Zubereitungen für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel i oder deren pharmazeutisch verträgliche Salze enthalten, können zum Beispiel oral, parenteral, intramuskulär, intravenös, rektal, nasal, durch Inhalation, subkutan oder durch eine geeignete transkutane Darreichungsform appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinär- als auch in der Humanmedizin und im Pflanzenschutz. Die Arzneimittel enthalten Wirkstoffe der Formel I und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen, perkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.
Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 30 mg/kg, vorzugsweise 1 mg/kg Körpergewicht. In akuten Situationen, etwa unmittelbar nach Erleiden apnoetischer Zustände im Hochgebirge, können auch noch höhere Dosen notwendig werden. Insbesondere bei i.v.-Anwendung, etwa bei einem Infarktpatienten auf der Intensivstation können bis zu 300 mg/kg pro Tag notwendig werden. Die Tagesdosis kann auf eine oder mehrere, zum Beispiel bis zu 4 Einzeldosen verteilt werden.

### Versuchsbeschreibungen und Beispiele

### Liste der verwendeten Abkürzungen:

- Fp.: schmelzpunkt
- min.: Minuten
- MPRC: Cartridge L-026-30; SI60 40-63µm; Super Vario Flash; max.press. 3 bar Götec-Labortechnik GmbH
- MPLC: Mitteldruck-Flüssigkeitschromatographie

### Beispiel 1: 2-Amino-4-(4-aminophenyl)-6-chlor-3,4-dihydrochinolin Hydrochlorid

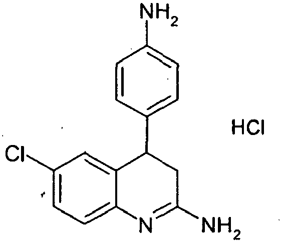

### a) 2-Amino-6-chlor-4-phenylchinolin

Eine unter Argonathmosphäre gerührte Suspension aus 1,4g gepulvertem KOH in 25ml wasserfreiem Acetonitril wurde 30 Minuten zum Sieden unter Rückflussbedingungen erhitzt und sodann mit einer Lösung aus 1,15 g 2-Amino-5-chlorbenzophenon in 5ml wasserfreiem Acetonitril versetzt. Die Mischung kochte 12 Stunden am Rückflusskühler. Nach dem Abkühlen goss man auf Eis, extrahierte mit Ethylacetat, wusch mit Wasser und destillierte das Lösungsmittel ab.
Fp.: 123-125°C.

### b) 2-Acetylamino-6-chlor-4-phenylchinolin

Eine Mischung aus 0,1 g 2-Amino-6-chlor-4-phenylchinolin und 8 ml Acetanhydrid wurde 1 Stunde bei 70°C gerührt, das Lösungsmittel abdestilliert, der feste Rückstand mit Wasser verrührt und die kristalline Substanz abfiltriert.
Fp.: 178-182°C.

### c) 2-Acetylamino-6-chlor-4-(4-nitrophenyl)-chinolin

200 mg 2-Acetylamino-6-chlor-4-phenylchinolin wurden bei 0°C portionsweise in 4ml 100%ige HNO₃ eingetragen, 30 min. bei 0°C bis 5°C gerührt und die Reaktionsmischung auf Eiswasser gegossen. Nach dem Neutralisieren mit 2n NaOH extrahierte man mit Methylacetat und destillierte das Lösungsmittel ab. Der feste Rückstand wurde mehrfach mit Diisopropylether ausgekocht und das Lösungsmittel des Filtrates so weit eingedampft, bis die Substanz sich kristallin abschied.
Fp.: ab 180°C unter Zersetzung.

### d) 2-Amino-6-chlor-4-(4-aminophenyl)-chinolin

Zu einer Suspension von 0,59 g 2-Acetylamino-6-chlor-4-(4-nitrophenyl)-chinolin und 0,29 g Eisenpulver tropfte man 1,8 ml konzentrierte Salzsäure, erhitzte sodann 4 Stunden am Rückfluss und filtrierte das heiße Reaktionsgemisch ab. Man destillierte das Lösungsmittel ab, führte eine MPLC auf einer MPRC-Kartusche mit einem Gemisch aus 10 Volumenteilen Dichlormethan und 1 Volumenteil Methanol durch und erhielt die Substanz als gelbes viskoses Öl.

### e) 2-Amino-4-(4-aminophenyl)-6-chlor-3,4-dihydrochinolin

Zu einer Lösung von 255mg 2-Amino-6-chlor-4-(4-aminophenyl)-chinolin in 3,4 ml Wasser und 22 ml Ethanol gab man im Verlauf einer Stunde unter Rührung 6,76g Natrium-Amalgam. Man rührte über Nacht, dekantierte vom Hg-Niederschlag und destillierte das Lösungsmittel unter vermindertem Druck. Nach Durchführung einer MPLC Säulenchromatographie in einem Gemisch aus 10 Volumenteilen Ethylacetat, 5 Volumenteilen Methanol, 5 Volumenteilen n-Heptan, 5 Volumenteilen Dichlormethan und 1 Volumenteil Ammoniak(konzentriert) stellte man mit wenig konzentrierter Salzsäure sauer, destillierte das Lösungsmittel ab und brachte den Rückstand unter wenig Ethylacetat zur Kristallisation.
Fp.: ab 300°C unter Zersetzung.

### Beispiel 2: 4-(4-Aminophenyl)-6-chlor-2-ethylamino-3,4-dihydrochinolin Hydrochlorid

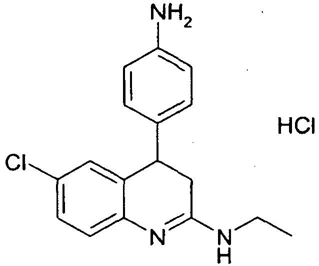

### a) 6-Chlor-2-ethylamino 4-(4-nitrophenyl)-chinolin

500 mg 2-Acetylamino-6-chlor-4-(4-nitrophenyl)-chinolin (Verbindung 1 c) wurden in 10 ml THF suspendiert und unter Argon portionsweise zu 2,9 ml Borane-THF Complex gegeben. Nach Aufschäumen entstand eine gelbe Lösung. Nach 12 Stunde Rühren bei Raumtemperatur, wurde die Lösung mit 5 ml Ethanol und 2 ml konzentrierter HCl versetzt und auf dem Wasserbad erwärmt, abkühlt und Wasser abdestilliert. Der Rückstand wurde mit Wasser versetzt, mit 2N NaOH alkalisch gestellt, mit Essigester extrahiert und mit Wasser gewaschen. Das Produkt wurde durch MPLC unter Verwendung einer MPRC-Kartusche mit einem Gemisch aus 1 Volumenteil Essigester, 2 Volumenteil Toluol chromatographisch gereinigt. Es wurde ein gelbes Festprodukt isoliert.
Fp.: 145-150°C.

### b) 4-(4-Aminophenyl)-6-chlor-2-ethylamino-chinolin

254 mg 6-Chlor-2-ethylamino 4-(4-nitrophenyl)-chinolin wurden in 1.6 ml Diglym suspendiert und mit 9 mg Eisenphthalocyanin und 97 mg 2-Bromethanol versetzt. Die Mischung wurde 3 Minuten bei Raumtemperatur gerührt und dann 3.1 ml NaBH₄-Lösung (0.5 M in Diglym) zuspritzt. Es fand eine exotherme Reaktion statt, die mit einem Wasserbad bei Raumtemperatur gehalten wurde. Die grünschwarze Mischung wurde 4 Stunden bei Raumtemperatur gerührt. Man versetzte die Mischung mit Wasser und extrahierte mit Essigester. Durch MPLC unter Verwendung einer MPRC-Kartusche mit einem Gemisch aus 1 Volumenteil Ethylacetat, und 1 Volumenteil Toluol wurde das Produkt chromatographisch gereinigt. Das Produkt wurde in wenig Essigester gelöst, mit etherischer HCl sauer gestellt, bei Raumtemperatur gerührt und abgesaugt.
Fp.: 308-314 °C

### c) 4-(4-Aminophenyl)-6-chlor-2-ethylamino-3,4-dihydrochinolin

148 mg 4-(4-Aminophenyl)-6-chlor-2-ethylamino-chinolin wurde in 10,3 ml Ethanol und 1,6 ml Wasser gelöst und innerhalb einer Stunde mit 3,6 g Natriumquecksilberamalgam versetzt. Man rührte über Nacht, dekantierte vom Hg-Niederschlag und destillierte das Lösungsmittel unter vermindertem Druck. Man stellte mit wenig konzentrierter Salzsäure sauer, destillierte das Lösungsmittel ab und brachte den Rückstand unter wenig Ethylacetat zur Kristallisation.
Fp.: ab 310°C unter Zersetzung.

### Beispiel 3: 4-(4-Aminophenyl)-6-chlor-2-diethylamino-3,4-dihydrochinolin Hydrochlorid

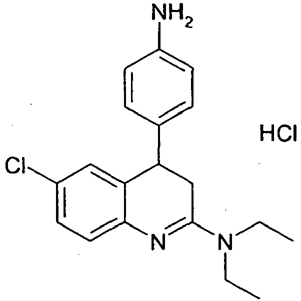

### a) 6-Chlor-2-(N-acetyl-N-ethylamino)-4-(4-nitrophenyl)-chinolin

600 mg 6-Chlor-2-ethylamino 4-(4-nitrophenyl)-chinolin (Verbindung 2a) wurden zu 0,19 g Acetanhydrid gegeben. Die Mischung wurde bei 70°C 4 Sunden gekocht, abgekühlt, und das Acetanhydrid abdistilliert. Der Rückstand wurde mit Wasser versetzt, mit Essigester extrahiert, Wasser gewaschen und das Produkt als gelbes Öl erhalten.

### b) 6-Chlor-2-diethylamino-4-(4-nitrophenyl)-chinolin

570 mg 6-Chlor-2-(N-acetyl-N-ethylamino)-4-(4-nitrophenyl)-chinolin wurden in 11,4 ml THF suspendiert und unter Argon portionsweise zu 3,1 ml Borane-THF-Komplex gegeben. Die Temperatur steigt auf 30°C. Nach 3 Stunde Rühren bei Raumtemperatur, wurde das Lösungsmittel abdestilliert und der Rückstand mit 50 ml Ethanol und 20 ml konzentrierter HCl versetzt und auf dem Wasserbad erwärmt, abkühlt und das Wasser abdestilliert. Der Rückstand wurde mit Wasser versetzt, mit 2N NaOH alkalisch gestellt, mit Essigester extrahiert und mit Wasser gewaschen. Das Produkt wurde durch MPLC unter Verwendung einer MPRC-Kartusche mit einem Gemisch aus 10 Volumenteilen Essigester, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Es wurde ein gelbes Festprodukt isoliert.
Fp.: 133-138°C.

### c) 4-(4-Aminophenyl)-6-chlor-2-diethylamino-chinolin

Zu einer Lösung von 380 mg 6-chlor-2-diethylamino-4-(4-nitrophenyl)-chinolin in 12,7 ml Eisessig gab man 179 mg Eisenpulver, tropfte anschließend 3,0 ml konzentrierte Salzsäure zu und kochte 2 Stunden bei 70°C. Man destillierte das Lösungsmittel ab, versetzte den Rückstand mit Wasser und stellte mit 2n NaOH alkalisch. Diese wässrige Phase wurde mit Ethylacetat extrahiert. Das Produkt ist ein dunkelbraunes, zähes Öl.

### d) 4-(4-Aminophenyl)-6-chlor-2-diethylamino-3,4-dihydrochinolin

Zu einer Lösung von 250 mg 4-(4-Aminophenyl)-6-chlor-2-diethylamino-chinolin in 2,5 ml Wasser und 16 ml Ethanol gab man im Verlauf von 5 Stunden unter Rühren 5,49 g Natriumquecksilberamalgam. Man rührte über Nacht, dekantierte vom Hg-Niederschlag und destillierte das Lösungsmittel unter vermindertem Druck. Der Rückstand wurde mit Wasser versetzt und mit Essigester extrahiert. Das Festprodukt als heller Reststoff wurde in wenig Essigester gelöst, mit etherischer HCl sauer gestellt, bei Raumtemperatur gerührt und abgesaugt.
Fp.: 128-134°C.

### Beispiel 4: N-(2-Dimethylaminoethyl)-N'-4-[(6-chlor-3,4-dihydro-2-diethylaminoquinolin-4-yl)phenyl]harnstoff Hydrochlorid

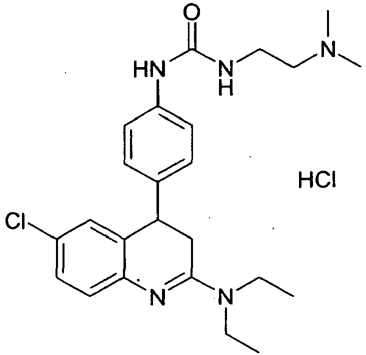

### a) O-(2-Nitrophenyl)-N-4-[(6-chlor-3,4-dihydro-2-diethylaminoquinolin-4-yl)phenyl] carbamate

62 mg 4-(4-Aminophenyl)-6-chlor-2-diethylamino-3,4-dihydrochinolin (Beispiel 3) wurde in 5 ml Dichlormethan gelöst und mit 46 mg 4-Nitrophenylchloroformat versetzt. Nach kurzer Zeit fällt ein Feststoff aus. Nach 3 Stunden Rühren bei Raumtemperatur destillierte man Dichlormethan ab. Das Produkt wurde isoliert als einen heller Feststoff.

### b) N-(2-Dimethylaminoethyl)-N'-[4-(6-chlor-3,4-dihydro-2-diethylaminoquinolin-4-yl)phenyl] harnstoff

105 mg O-(2-Nitrophenyl)-N-[4-(6-chloro-3,4-dihydro-2-diethylaminoquinolin-4-yl)phenyl] carbamate wurden in 5 ml THF gelöst. Man tropfte anschließend 21 mg N,N-Dimethylethylenediamine zu und rührte 4 Stunden bei Raumtemperatur. Das Lösungsmittel wurde abdestilliert und der Rückstand wurde mit Wasser und Essigester extrahiert. Das Produkt wurde durch MPLC unter Verwendung einer MPRC-Kartusche mit einem Gemisch aus 10 Volumenteilen Essigester, 5 Volumenteilen n-Heptan, 5 Volumenteilen Dichlormethan, 5 Volumenteilen Methanol und 1 Volumenteil konzentrierter wässriger Ammoniaklösung chromatographisch gereinigt. Das erhaltene Produkt wurde mit Wasser, 2N HCl und 10% HCl versetzt und die klare Lösung wurde über Nacht gefriergetrocknet. Man erhielt das Produkt als ein hygroskopisches Festprodukt dass in wenig Essigester gelöst wurde, mit etherischer HCl sauer gestellt, bei Raumtemperatur gerührt und abgesaugt.
Rf = 0.57 (silica gel, Essigester/Methanol/Heptan/Dichloromethan/Ammoniak 10:5:5:5:1)
1H-NMR (DMSO-d6): δ = 1.07(t,2H), 1.17(t,2H), 2.50(s,6H), 2.80(d,2H), 3.13(q,2H), 3.30(m,1H), 3.57(m,1H), 3.70(m,1H), 3.83(q,2H), 4.39(m,1H), 6.66(t,1H), 6.99(d,1H), 7.04(d,2H), 7.43(m,3H), 7.70(d,1 H), 9.11 (s, 1 H)
MS: m/z = 441 (m)+

### Pharmakologische Daten:

### Testbeschreibung: Bestimmung der NHE-inhibitorischen Wirkung

In diesem Test wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wurde die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl -Puffer: 115 mM NaCl, 20 mM NH₄Cl, 5 mM KCl, 1 mM CaCl₂, 1 mM MgSO₄, 20 mM Hepes, 5 mM Glucose, 1 mg/ml BSA; mit 1 M NaOH wurde ein pH von 7,4 eingestellt). Die intrazelluläre Ansäuerung wurde durch Zugabe von 975 µl eines NH₄Cl -freien Puffers (s. u.) zu 25 µl Aliquots der in NH₄Cl - Puffer inkubierten Zellen induziert. Die nachfolgende Geschwindigkeit der pH-Erholung wurde bei NHE1 zwei Minuten, bei NHE2 fünf Minuten und bei NHE3 drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCl, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wurde ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wurde ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

Die inhibitorische Wirkung (IC₅₀-Werte) diverser Beispielverbindungen auf den NHE3 ist in nachfolgender Tabelle aufgeführt:

| Beispiel | IC₅₀ [µM] |
|---|---|
| 1 | 4,39 |
| 2 | 10,87 |
| 3 | 9,19 |
| 4 | 13,85 |

## Patentansprüche

1. Verbindungen der Formel I 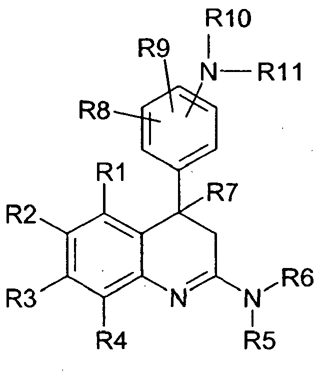 worin bedeuten:
R1, R2, R3 und R4
unabhängig voneinander Wasserstoff, F, Cl, Br, I, CN, NO₂, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6
bilden gemeinsam mit dem Stickstoffatom, an das sie gebunden sind, einen 4-, 5-, 6-, 7-, 8-, 9- oder 10-gliedrigen Ring, in dem ein oder zwei CH₂-Gruppen unabhängig voneinander durch NR12, Schwefel, Sauerstoff, C(O) oder SO₂ ersetzt sein können;
R12 Wasserstoff, Alkyl mit 1,2,3 oder 4 C-Atomen oder Cycloalkyl mit 3, 4, 5 oder 6 C-Atomen;
R7 Wasserstoff oder Alkyl mit 1, 2, 3 oder 4 C-Atomen;
R8 und R9
unabhängig voneinander Wasserstoff, F, Cl, Br, OH, Alkyl mit 1, 2, 3 oder 4 C-Atomen, CH₃O, CF₃, oder CH₃SO₂;
R10 und R11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl), 1-Morpholinyl -COOR15, OR16, NR17R18 oder Phenyl, das unabhängig voneinander 1 oder 2 Substituenten trägt, ausgewählt aus der Gruppe Chlor, Fluor, Methyl und Methoxy;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem ein, zwei oder drei CH₂-Gruppen unabhängig voneinander durch NR19, Schwefel, Sauerstoff, C(O) oder SO₂ ersetzt sein können;
R19 Wasserstoff, Alkyl mit 1, 2, 3 oder 4 C-Atomen oder Cycloalkyl mit 3, 4, 5 oder 6 C-Atomen;
sowie deren pharmazeutisch verträgliche Salze und Trifluoracetate.

2. Verbindungen der Formel I nach Anspruch 1, worin bedeuten
R1, R2, R3 und R4
unabhängig voneinander Wasserstoff, F, Cl, Br, CN, CF₃, CH₃-SO₂, Alkyl mit 1, 2, 3 oder 4 C-Atomen, NH₂, NH-CH₃ oder N(CH₃)₂;
R5 und R6
unabhängig voneinander Wasserstoff, Alkyl mit 1, 2, 3, 4, 5 oder 6 C-Atomen, CF₃-CH₂-, Cycloalkyl mit 3, 4, 5 oder 6 C-Atomen oder Cyclopropyl-CH₂-,
oder
R5 und R6
bilden gemeinsam mit dem Stickstoffatom, an das sie gebunden sind, einen 4-, 5-, 6-, 7-, 8-, 9- oder 10-gliedrigen Ring;
R7 Wasserstoff;
R8 und R9
unabhängig voneinander Wasserstoff, F, Cl, OH, Alkyl mit 1, 2, 3 oder 4 C-Atomen, CH₃O, CF₃, oder CH₃SO₂;
R10 und 11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Alkyl mit 1, 2, 3 oder 4 C-Atomen, Cycloalkyl mit 3, 4, 5 oder 6 C-Atomen, 1-Pyrrolidinyl, 1-Piperidinyl, 1-(4-Methylpiperazinyl) 1-Morpholinyl, -COOR15, OR16, NR17R18 oder Phenyl, das unabhängig voneinander 1 oder 2 Substituenten trägt, ausgewählt aus der Gruppe Chlor, Fluor, Methyl und Methoxy;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch Sauerstoff oder NR19 ersetzt sein kann;
R19 Wasserstoff oder Alkyl mit 1, 2, 3 oder 4 C-Atomen;
sowie deren pharmazeutisch verträgliche Salze und Trifluoracetate.

3. Verbindungen der Formel I nach Anspruch 1 oder 2, worin bedeuten
R1, R2, R3 und R4
unabhängig voneinander Wasserstoff, F, Cl, Br, CN, CF₃, CH₃-SO₂, Methyl, Ethyl, NH₂, NH-CH₃ oder N(CH₃)₂;
R5 und R6
unabhängig voneinander Wasserstoff, Methyl, Ethyl, Isopropyl, CF₃-CH₂ oder Cycloalkyl mit 3, 4, 5 oder 6 C-Atomen;
R7 Wasserstoff;
R8 und R9
unabhängig voneinander Wasserstoff, Cl oder Methyl;
R10 und 11
unabhängig voneinander R13-(CₘH₂ₘ)-Bₙ, wobei
m Null, 1, 2, 3 oder 4;
n Null oder 1;
B -CO-, -CONR14- oder -SO₂-;
R14 Wasserstoff oder Alkyl mit 1, 2, 3, 4, 5 oder 6 C-Atomen;
R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch Sauerstoff oder NR19 ersetzt sein kann;
R19 Wasserstoff oder Methyl;
sowie deren pharmazeutisch verträgliche Salze und Trifluoracetate.

4. Verbindungen der Formel I nach einem oder mehreren der Ansprüche 1 bis 3, worin bedeuten
R1 und R3
Wasserstoff;
R2 und R4
unabhängig voneinander Wasserstoff oder Cl;
R5 und R6
unabhängig voneinander Wasserstoff, Methyl oder Ethyl;
R7 Wasserstoff;
R8 und R9
unabhängig voneinander Wasserstoff oder Cl;
R10 und 11
unabhängig voneinander R13-(CₘH₂ₘ)-Bₙ, wobei
m Null, 1, 2, 3 oder 4;
n Null oder 1;
B -CONR14-;
R14 Wasserstoff oder Methyl;
R13 Wasserstoff, Methyl, Cycloalkyl mit 3, 4, 5 oder 6 C-Atomen, 1-Pyrrolidinyl, 1-Piperidinyl, 1-(4-Methylpiperazinyl), -COOR15, OR16 oder NR17R18;
R15, R16, R17 und R18
unabhängig voneinander Wasserstoff oder Alkyl mit 1, 2, 3, 4, 5 oder 6 C-Atomen;
oder
R10 und R11
bilden gemeinsam mit dem Stickstoffatom, an das sie gebunden sind, einen 4-, 5-, 6-, 7-, 8-, 9- oder 10-gliedrigen Ring, in dem eine CH₂-Gruppen durch NR19 ersetzt sein kann;
R19 Wasserstoff oder Methyl;
sowie deren pharmazeutisch verträgliche Salze und Trifluoracetate.

5. Verbindungen der Formel I nach einem oder mehreren der Ansprüche 1 bis 4, ausgewählt aus der Gruppe:
2-Amino-4-(4-aminophenyl)-6-chlor-3,4-dihydrochinolin,
4-(4-Aminophenyl)-6-chlor-2-ethylamino-3,4-dihydrochinolin,
4-(4-Aminophenyl)-6-chlor-2-diethylamino-3,4-dihydrochinolin
und
N-(2-Dimethylaminoethyl)-N'-4-[(6-chloro-3,4-dihydro-2-diethylaminoquinolin-4-yl)phenyl]harnstoff,
sowie dessen pharmazeutisch verträgliche Salze und Trifluoracetate.

6. Verbindungen der Formel I und deren pharmazeutisch verträgliche Salze nach einem oder mehreren der Ansprüche 1 bis 5 zur Verwendung als Medikament.

7. Verwendung einer Verbindung der Formel I und/oder dessen pharmazeutisch verträglicher Salze nach einem oder mehreren der Ansprüche 1 bis 5 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, degenerative Erkrankungen des ZNS, Verminderung der Gedächtnisleistung, Demens und Alzheimersche Erkrankung, und von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt, zur Konservierung und Lagerung von Transplantaten für chirurgische Maßnahmen, für den Einsatz bei chirurgischen Operationen und Organtransplantationen und zur Gesunderhaltung und Lebensverlängerung.

8. Verwendung einer Verbindung der Formel I und/oder dessen pharmazeutisch verträglicher Salze nach einem oder mehreren der Ansprüche der Ansprüche 1 bis 5 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, von Schockzuständen oder der Zuckerkrankheit und diabetischer Spätschäden oder von Krankheiten, bei denen die Zellproliferation eine primäre oder sekundäre Ursache darstellt zur Konservierung und Lagerung von Transplantaten für chirurgische Maßnahmen, für den Einsatz bei chirurgischen Operationen und Organtransplantationen und zur Gesunderhaltung und Lebensverlängerung.

9. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

10. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe des Schnarchens.

11. Verwendung einer Verbindung der Formol **I** und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

12. Verwendung einer Verbindung der Formel I und/oder dessen pharmazeutisch verträglicher Salze nach einem oder mehreren der Ansprüche 1 bis 5 allein oder in Kombination mit anderen Arzneimitteln oder Wirkstoffen zur Herstellung eines Medikaments zur Behandlung oder Prophylaxe von Störungen der Darmfunktion.

13. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 5.

14. Pharmazeutische Zubereitung für die humane, veterinäre oder phytoprotektive Anwendung enthaltend eine wirksame Menge einer Verbindung der Formel I und/oder eines pharmazeutisch verträglichen Salzes davon nach einem oder mehreren der Ansprüche 1 bis 5, in Kombination mit anderen pharmakologischen Wirkstoffen oder Arzneimitteln.

## Claims

1. A compound of the formula I 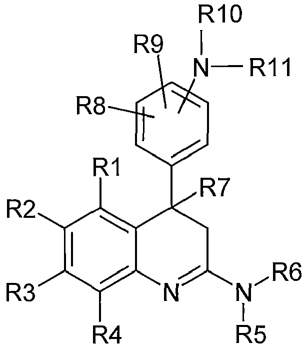 in which the meanings are:
R1, R2, R3 and R4
independently of one another hydrogen, F, Cl, Br, I, CN, NO₂, CF₃, CH₃-SO₂, alkyl having 1, 2, 3 or 4 C-atoms, NH₂, NH-CH₃ or N(CH₃)₂;
R5 and R6
independently of one another hydrogen, alkyl having 1, 2, 3, 4, 5 or 6 C atoms, CF₃-CH₂-, cycloalkyl having 3, 4, 5 or 6 C-atoms or cyclopropyl-CH₂-,
or
R5 and R6
form together with the nitrogen atom to which they are bonded a 4-, 5-, 6-, 7-, 8-, 9- or 10-membered ring in which one or two CH₂ groups may be replaced independently of one another by NR12, sulfur, oxygen, C(O) or SO₂;
R12 hydrogen, alkyl having 1, 2, 3 or 4 C-atoms or cycloalkyl having 3, 4, 5 or 6 C-atoms;
R7 hydrogen or alkyl having 1, 2, 3 or 4 C-atoms;
R8 and R9
independently of one another hydrogen, F, Cl, Br, OH, alkyl having 1, 2, 3 or 4 C-atoms, CH₃O, CF₃, or CH₃SO₂;
R10 and R11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C-atoms;
R13 hydrogen, alkyl having 1, 2, 3 or 4 C-atoms, cycloalkyl having 3, 4, 5 or 6 C-atoms, 1-pyrrolidinyl, 1-piperidinyl, 1-(4-methylpiperazinyl), 1-morpholinyl, -COOR15, OR16, NR17R18 or phenyl which has independently of one another 1 or 2 substituents selected from the group of chlorine, fluorine, methyl and methoxy;
R15, R16, R17 and R18
independently of one another hydrogen or alkyl having 1, 2, 3, 4, 5 or 6 C-atoms;
or
R10 and R11
form together with the nitrogen atom to which they are bonded a 4-, 5-, 6-, 7-, 8-, 9- or 10-membered ring in which one, two or three CH₂ groups may be replaced independently of one another by NR19, sulfur, oxygen, C(O) or SO₂;
R19 hydrogen, alkyl having 1, 2, 3 or 4 C-atoms or cycloalkyl having 3, 4, 5 or 6 C-atoms;
and the pharmaceutically acceptable salts and trifluoroacetates thereof.

2. A compound of the formula I as claimed in claim 1, in which the meanings are
R1, R2, R3 and R4
independently of one another hydrogen, F, Cl, Br, CN, CF₃, CH₃-SO₂, alkyl having 1, 2, 3 or 4 C-atoms, NH₂, NH-CH₃ or N(CH₃)₂;
R5 and R6
independently of one another hydrogen, alkyl having 1, 2, 3, 4, 5 or 6 C-atoms, CF₃-CH₂-, cycloalkyl having 3, 4, 5 or 6 C-atoms or cyclopropyl-CH₂-,
or
R5 and R6
form together with the nitrogen atom to which they are bonded a 4-, 5-, 6-, 7-, 8-, 9- or 10-membered ring;
R7 hydrogen;
R8 and R9
independently of one another hydrogen, F, Cl, OH, alkyl having 1, 2, 3 or 4 C-atoms, CH₃O, CF₃, or CH₃SO₂;
R10 and 11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C-atoms;
R13 hydrogen, alkyl having 1, 2, 3 or 4 C-atoms, cycloalkyl having 3, 4, 5 or 6 C-atoms, 1-pyrrolidinyl, 1-piperidinyl, 1-(4-methylpiperazinyl), 1-morpholinyl, -COOR15, OR16, NR17R18 or phenyl which has independently of one another 1 or 2 substituents selected from the group of chlorine, fluorine, methyl and methoxy;
R15, R16, R17 and R18
independently of one another hydrogen or alkyl having 1, 2, 3, 4, 5 or 6 C-atoms;
or
R10 and R11
form together with the nitrogen atom to which they are bonded a 4-, 5-, 6-, 7-, 8-, 9- or 10-membered ring in which one CH₂ group may be replaced by oxygen or NR19;
R19 hydrogen or alkyl having 1, 2, 3 or 4 C-atoms;
and the pharmaceutically acceptable salts and trifluoroacetates thereof.

3. A compound of the formula I as claimed in claim 1 or 2, in which the meanings are:
R1, R2, R3 and R4
independently of one another hydrogen, F, Cl, Br, CN, CF₃, CH₃-SO₂,
methyl, ethyl, NH₂, NH-CH₃ or N(CH₃)₂;
R5 and R6
independently of one another hydrogen, methyl, ethyl, isopropyl, CF₃-CH₂ or cycloalkyl having 3, 4, 5 or 6 C-atoms;
R7 hydrogen;
R8 and R9
independently of one another hydrogen, Cl or methyl;
R10 and 11
independently of one another R13-(CₘH₂ₘ)-Bₙ, where
m zero, 1, 2, 3 or 4;
n zero or 1;
B -CO-, -CONR14- or -SO₂-;
R14 hydrogen or alkyl having 1, 2, 3, 4, 5 or 6 C-atoms;
R13 hydrogen, methyl, cycloalkyl having 3, 4, 5 or 6 C-atoms, 1-pyrrolidinyl, 1-piperidinyl, 1-(4-methylpiperazinyl), -COOR15, OR16 or NR17R18;
R15, R16, R17 and R18
independently of one another hydrogen or alkyl having 1, 2, 3, 4, 5 or 6 C-atoms;
or
R10 and R11
form together with the nitrogen atom to which they are bonded a 4-, 5-, 6-, 7-, 8-, 9- or 10-membered ring, in which one CH₂ group may be replaced by oxygen or NR19;
R19 hydrogen or methyl;
and the pharmaceutically acceptable salts and trifluoroacetates thereof.

4. A compound of the formula I as claimed in one or more of claims 1 to 3, in which the meanings are
R1 and R3
hydrogen;
R2 and R4
independently of one another hydrogen or Cl;
R5 and R6
independently of one another hydrogen, methyl or ethyl;
R7 hydrogen;
R8 and R9
independently of one another hydrogen or Cl;
R10 and 11
independently of one another R13-(CₘH₂ₘ)-Bₙ, where
m zero, 1, 2, 3 or 4;
n zero or 1;
B -CONR14-;
R14 hydrogen or methyl;
R13 hydrogen, methyl, cycloalkyl having 3, 4, 5 or 6 C-atoms, 1-pyrrolidinyl, 1-piperidinyl, 1-(4-methylpiperazinyl), -COOR15, OR16 or NR17R18;
R15, R16, R17 and R18
independently of one another hydrogen or alkyl having 1, 2, 3, 4, 5 or 6 C-atoms;
or
R10 and R11
form together with the nitrogen atom to which they are bonded a 4-, 5-, 6-, 7-, 8-, 9- or 10-membered ring in which one CH₂ group may be replaced by NR19;
R19 hydrogen or methyl;
and the pharmaceutically acceptable salts and trifluoroacetates thereof.

5. A compound of the formula I as claimed in one or more of claims 1 to 4, selected from the group:
2-amino-4-(4-aminophenyl)-6-chloro-3,4-dihydroquinoline,
4-(4-aminophenyl)-6-chloro-2-ethylamino-3,4-dihydroquinoline,
4-(4-aminophenyl)-6-chloro-2-diethylamino-3,4-dihydroquinoline
and
N-(2-dimethylaminoethyl)-N'-4-[(6-chloro-3,4-dihydro-2-diethylaminoquinolin-4-yl)phenyl]urea,
and the pharmaceutically acceptable salts and trifluoroacetates thereof.

6. A compound of the formula I and the pharmaceutically acceptable salts thereof as claimed in one or more of claims 1 to 5 for use as a medicament.

7. The use of a compound of the formula I and/or its pharmaceutically acceptable salts as claimed in one or more of claims 1 to 5 for the manufacture of a medicament for the treatment or prophylaxis of impairments of respiratory drive, of respiratory disorders, sleep-related respiratory disorders, sleep apneas, of snoring, of acute and chronic renal disorders, of acute renal failure and of chronic renal failure, of impairments of bowel function, of high blood pressure, of essential hypertension, of central nervous system disorders, of disorders resulting from CNS overexcitability, epilepsy and centrally induced spasms or of anxiety states, depressions and psychoses, of ischemic states of the peripheral or central nervous system or of stroke, degenerative CNS disorders, reduced memory capacity, dementia and Alzheimer's disease, and of acute and chronic damage and disorders of peripheral organs or limbs caused by ischemic or reperfusion events, of atherosclerosis, of impairments of lipid metabolism, of thromboses, of impairments of biliary function, of infestation by ectoparasites, of disorders resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

8. The use of a compound of the formula I and/or its pharmaceutically acceptable salts as claimed in one or more of claims 1 to 5 in combination with other pharmaceuticals or active ingredients for the manufacture of a medicament for the treatment or prophylaxis of impairments of respiratory drive, of respiratory disorders, sleep-related respiratory disorders, sleep apneas, of snoring, of acute and chronic renal disorders, of acute renal failure and of chronic renal failure, of impairments of bowel function, of high blood pressure, of essential hypertension, of central nervous system disorders, of disorders resulting from CNS overexcitability, epilepsy and centrally induced spasms or of anxiety states, depressions and psychoses, of ischemic states of the peripheral or central nervous system or of stroke, of acute and chronic damage and disorders of peripheral organs or limbs caused by ischemic or reperfusion events, of atherosclerosis, of impairments of lipid metabolism, of thromboses, of impairments of biliary function, of infestation by ectoparasites, of disorders resulting from endothelial dysfunction, of protozoal diseases, of malaria, of states of shock or of diabetes and late damage from diabetes or of diseases in which cell proliferation represents a primary or secondary cause, for the preservation and storage of transplants for surgical procedures, for use in surgical operations and organ transplantations and for maintaining health and prolonging life.

9. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of impairments of the respiratory drive and/or of sleep-related respiratory impairments such as sleep apneas.

10. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of snoring.

11. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of acute or chronic renal disorders, of acute renal failure or of chronic renal failure.

12. The use of a compound of the formula I and/or its pharmaceutically acceptable salts as claimed in one or more of claims 1 to 5 alone or in combination with other pharmaceuticals or active ingredients for the manufacture of a medicament for the treatment or prophylaxis of impairments of bowel function.

13. A pharmaceutical preparation for human, veterinary or phytoprotective use comprising an effective amount of a compound of the formula I and/or of a pharmaceutically acceptable salt thereof as claimed in one or more of claims 1 to 5.

14. A pharmaceutical preparation for human, veterinary or phytoprotective use comprising an effective amount of a compound of the formula I and/or of a pharmaceutically acceptable salt thereof as claimed in one or more of claims 1 to 5, in combination with other pharmacological active ingredients or pharmaceuticals.

## Revendications

1. Composés de formule I 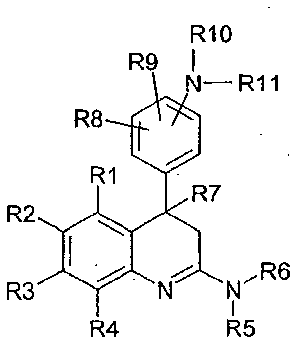 dans laquelle :
R1, R2, R3 et R4 signifient, indépendamment les uns des autres, hydrogène, F, Cl, Br, I, CN, NO₂, CF₃, CH₃-SO₂, alkyle comprenant 1, 2, 3 ou 4 atomes de carbone, NH₂, NH-CH₃ ou N(CH₃)₂ ;
R5 et R6 signifient, indépendamment l'un de l'autre hydrogène, alkyle comprenant 1, 2, 3, 4, 5 ou 6 atomes de carbone, CF₃-CH₂-, cycloalkyle comprenant 3, 4, 5 ou 6 atomes de carbone ou cyclopropyl-CH₂-ou
R5 et R6 forment ensemble avec l'atome d'azote auquel ils sont liés, un cycle de 4, 5, 6, 7, 8, 9 ou 10 chaînons, dans lequel un ou deux groupes CH₂ peuvent être remplacés, indépendamment l'un de l'autre, par NR12, soufre, oxygène, C(O) ou SO₂ ; R12 signifie hydrogène, alkyle comprenant 1, 2, 3 ou 4 atomes de carbone ou cycloalkyle comprenant 3, 4, 5 ou 6 atomes de carbone ;
R7 signifie hydrogène ou alkyle comprenant 1, 2, 3 ou 4 atomes de carbone ;
R8 et R9 signifient, indépendamment l'un de l'autre, hydrogène, F, Cl, Br, OH, alkyle comprenant 1, 2, 3 ou 4 atomes de carbone, CH₃O, CF₃, ou CH₃SO₂ ;
R10 et R11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, alkyle comprenant 1, 2, 3 ou 4 atomes de carbone, cycloalkyle comprenant 3, 4, 5 ou 6 atomes de carbone, 1-pyrrolidinyle, 1-pipéridinyle, 1-(4-méthylpipérazinyle), 1-morpholinyle, -COOR15, OR16, NR17R18 ou phényle, qui porte, indépendamment l'un de l'autre, 1 ou 2 substituants, choisis dans le groupe chlore, fluor, méthyle et méthoxy ;
R15, R16, R17 et R18 signifient indépendamment les uns des autres, hydrogène ou alkyle comprenant 1, 2, 3, 4, 5 ou 6 atomes de carbone ; ou
R10 et R11 forment ensemble avec l'atome d'azote auquel ils sont liés, un cycle de 4, 5, 6, 7, 8, 9 ou 10 chaînons, dans lequel un, deux ou trois groupes CH₂ peuvent être remplacés, indépendamment les uns des autres, par NR19, soufre, oxygène, C(O) ou SO₂ ;
R19 signifie hydrogène, alkyle comprenant 1, 2, 3 ou 4 atomes de carbone ou cycloalkyle comprenant 3, 4, 5 ou 6 atomes de carbone ;
ainsi que leurs sels pharmaceutiquement acceptables et leurs trifluoroacétates.

2. Composés de formule I selon la revendication 1, dans laquelle :
R1, R2, R3 et R4 signifient, indépendamment les uns des autres, hydrogène, F, Cl, Br, CN, CF₃, CH₃-SO₂, alkyle comprenant 1, 2, 3 ou 4 atomes de carbone, NH₂, NH-CH₃ ou N(CH₃)₂ ;
R5 et R6 signifient, indépendamment l'un de l'autre hydrogène, alkyle comprenant 1, 2, 3, 4, 5 ou 6 atomes de carbone, CF₃-CH₂-, cycloalkyle comprenant 3, 4, 5 ou 6 atomes de carbone ou cyclopropyl-CH₂-ou
R5 et R6 forment ensemble, avec l'atome d'azote auquel ils sont liés, un cycle de 4, 5, 6, 7, 8, 9 ou 10 chaînons ;
R7 signifie hydrogène ;
R8 et R9 signifient, indépendamment l'un de l'autre, hydrogène, F, Cl, OH, alkyle comprenant 1, 2, 3 ou 4 atomes de carbone, CH₃O, CF₃, ou CH₃SO₂ ;
R10 et 11 signifient, indépendamment l'un de l'autre, R13- (CₘH₂ₘ) -Bn, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, alkyle comprenant 1, 2, 3 ou 4 atomes de carbone, cycloalkyle comprenant 3, 4, 5 ou 6 atomes de carbone, 1-pyrrolidinyle, 1-pipéridinyle, 1-(4-méthylpipérazinyle), 1-morpholinyle, -COOR15, OR16, NR17R18 ou phényle, qui porte, indépendamment l'un de l'autre, 1 ou 2 substituants, choisis dans le groupe chlore, fluor, méthyle et méthoxy ;
R15, R16, R17 et R18 signifient, indépendamment les uns des autres, hydrogène ou alkyle comprenant 1, 2, 3, 4, 5 ou 6 atomes de carbone ; ou
R10 et R11 forment ensemble avec l'atome d'azote auquel ils sont liés, un cycle de 4, 5, 6, 7, 8, 9 ou 10 chaînons, dans lequel un groupe CH₂ peut être remplacé par oxygène ou NR19 ;
R19 signifie hydrogène ou alkyle comprenant 1, 2, 3 ou 4 atomes de carbone ;
ainsi que leurs sels pharmaceutiquement acceptables et leurs trifluoroacétates.

3. Composés de formule I selon la revendication 1 ou 2, où
R1, R2, R3 et R4 signifient, indépendamment les uns des autres, hydrogène, F, Cl, Br, CN, CF₃, CH₃-SO₂, méthyle, éthyle, NH₂, NH-CH₃ ou N(CH₃)₂ ;
R5 et R6 signifient, indépendamment l'un de l'autre, hydrogène, méthyle, éthyle, isopropyle, CF₃-CH₂ ou cycloalkyle comprenant 3, 4, 5 ou 6 atomes de carbone ;
R7 signifie hydrogène ;
R8 et R9 signifient, indépendamment l'un de l'autre hydrogène, Cl ou méthyle ;
R10 et 11 signifient, indépendamment l'un de l'autre, R13- (CₘH₂ₘ)-Bₙ, où
m vaut zéro, 1, 2, 3 ou 4 ;
n vaut zéro ou 1 ;
B signifie -CO-, -CONR14- ou -SO₂- ;
R14 signifie hydrogène ou alkyle comprenant 1, 2, 3, 4, 5 ou 6 atomes de carbone ;
R13 signifie hydrogène, méthyle, cycloalkyle comprenant 3, 4, 5 ou 6 atomes de carbone, 1-pyrrolidinyle, 1-pipéridinyle, 1-(4-méthylpipérazinyle), -COOR15, OR16 ou NR17R18 ;
R15, R16, R17 et R18 signifient, indépendamment les uns des autres, hydrogène ou alkyle comprenant 1, 2, 3, 4, 5 ou 6 atomes de carbone ; ou
R10 et R11 forment ensemble avec l'atome d'azote auquel ils sont liés, un cycle de 4, 5, 6, 7, 8, 9 ou 10 chaînons, dans lequel un groupe CH₂ peut être remplacé par oxygène ou NR19 ;
R19 signifie hydrogène ou méthyle ;
ainsi que leurs sels pharmaceutiquement acceptables et leurs trifluoroacétates.

4. Composés de formule I selon l'une ou plusieurs des revendications 1 à 3, où
R1 et R3 signifient hydrogène ;
R2 et R4 signifient, indépendamment l'un de l'autre hydrogène ou Cl ;
R5 et R6 signifient, indépendamment l'un de l'autre, hydrogène, méthyle ou éthyle ;
R7 signifie hydrogène ;
R8 et R9 signifient, indépendamment l'un de l'autre hydrogène ou Cl ;
R10 et 11 signifient, indépendamment l'un de l'autre, R13-(CₘH₂ₘ)-Bₙ, où
m vaut zéro, 1, 2, 3 ou 4 ;
n vaut zéro ou 1 ;
B signifie -CONR14- ;
R14 signifie hydrogène ou méthyle ;
R13 signifie hydrogène, méthyle, cycloalkyle comprenant 3, 4, 5 ou 6 atomes de carbone, 1-pyrrolidinyle, 1-pipéridinyle, 1-(4-méthylpipérazinyle), -COOR15, OR16 ou NR17R18 ;
R15, R16, R17 et R18 signifient, indépendamment les uns des autres, hydrogène ou alkyle comprenant 1, 2, 3, 4, 5 ou 6 atomes de carbone ; ou
R10 et R11 forment ensemble avec l'atome d'azote auquel ils sont liés, un cycle de 4, 5, 6, 7, 8, 9 ou 10 chaînons, dans lequel un groupe CH₂ peut être remplacé NR19 ;
R19 signifie hydrogène ou méthyle ;
ainsi que leurs sels pharmaceutiquement acceptables et leurs trifluoroacétates.

5. Composés de formule I selon l'une ou plusieurs des revendications 1 à 4, choisis dans le groupe formé par
la 2-amino-4-(4-aminophényl)-6-chloro-3,4-dihydroquinoléine,
la 4-(4-aminophényl)-6-chloro-2-éthylamino-3,4-dihydroquinoléine,
la 4-(4-aminophényl)-6-chloro-2-diéthylamino-3,4-dihydroquinoléine et
la N-(2-diméthylaminoéthyl)-N'-4-[(6-chloro-3,4-dihydro-2-diéthylaminoquinoléin-4-yl)phényl]urée,
ainsi que leurs sels pharmaceutiquement acceptables et leurs trifluoroacétates.

6. Composés de formule I et leurs sels pharmaceutiquement acceptables selon l'une ou plusieurs des revendications 1 à 5 pour une utilisation comme médicament.

7. Utilisation d'un composé de formule I et/ou de ses sels pharmaceutiquement acceptables selon l'une ou plusieurs des revendications 1 à 5 pour la préparation d'un médicament pour le traitement ou la prophylaxie de troubles de l'effort respiratoire, de troubles respiratoires, de troubles respiratoires provoqués par le sommeil, d'apnées du sommeil, de ronflements, de maladies rénales aiguës et chroniques, de l'insuffisance rénale aiguë et de l'insuffisance rénale chronique, de troubles de la fonction intestinale, de l'hypertension, de l'hypertonie essentielle, de maladies du système nerveux central, de maladies qui résultent d'une hyperexcitabilité du système nerveux central, de l'épilepsie et de crampes déclenchées de manière centrale ou d'états d'anxiété, de dépressions et de psychoses, d'états ischémiques du système nerveux périphérique et central ou d'un accident vasculaire cérébral, de maladies dégénératives du système nerveux central, de la diminution de la mémoire, de la démence et de la maladie d'Alzheimer et de lésions et de maladies aiguës et chroniques d'organes périphériques ou de membres, qui sont provoquées par des événements d'ischémie ou de reperfusion, de l'athérosclérose, de troubles du métabolisme, de thromboses, de troubles de la fonction biliaire, d'une attaque par des ectoparasites, de maladies suite à un dysfonctionnement endothélial, de maladies protozoaires, de la malaria, d'états de choc ou du diabète et de lésions tardives diabétiques ou de maladies dans lesquelles la prolifération cellulaire représente une cause primaire ou secondaire, pour la conservation et l'entreposage de greffes pour des interventions chirurgicales, pour l'utilisation lors d'opérations chirurgicales et de transplantations d'organes et pour le maintien de la santé et le prolongement de la vie.

8. Utilisation d'un composé de formule I et/ou de ses sels pharmaceutiquement acceptables selon l'une ou plusieurs des revendications 1 à 5 en combinaison avec d'autres médicaments ou substances actives pour la préparation d'un médicament pour le traitement ou la prophylaxie de troubles de l'effort respiratoire, de troubles respiratoires, de troubles respiratoires provoqués par le sommeil, d'apnées du sommeil, de ronflements, de maladies rénales aiguës et chroniques, de l'insuffisance rénale aiguë et de l'insuffisance rénale chronique, de troubles de la fonction intestinale, de l'hypertension, de l'hypertonie essentielle, de maladies du système nerveux central, de maladies qui résultent d'une hyperexcitabilité du système nerveux central, de l'épilepsie et de crampes déclenchées de manière centrale ou d'états d'anxiété, de dépressions et de psychoses, d'états ischémiques du système nerveux périphérique et central ou d'un accident vasculaire cérébral, de maladies dégénératives du système nerveux central, de la diminution de la mémoire, de la démence et de la maladie d'Alzheimer et de lésions et de maladies aiguës et chroniques d'organes périphériques ou de membres, qui sont provoquées par des événements d'ischémie ou de reperfusion, de l'athérosclérose, de troubles du métabolisme, de thromboses, de troubles de la fonction biliaire, d'une attaque par des ectoparasites, de maladies suite à un dysfonctionnement endothélial, de maladies protozoaires, de la malaria, d'états de choc ou du diabète et de lésions tardives diabétiques ou de maladies dans lesquelles la prolifération cellulaire représente une cause primaire ou secondaire, pour la conservation et l'entreposage de greffes pour des interventions chirurgicales, pour l'utilisation lors d'opérations chirurgicales et de transplantations d'organes et pour le maintien de la santé et le prolongement de la vie.

9. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie de troubles de l'effort respiratoire et/ou de troubles respiratoires provoqués par le sommeil, tels que les apnées du sommeil.

10. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie du ronflement.

11. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie de maladies rénales aiguës ou chroniques, de l'insuffisance rénale aiguë ou de l'insuffisance rénale chronique.

12. Utilisation d'un composé de formule I et/ou de ses sels pharmaceutiquement acceptables selon l'une ou plusieurs des revendications 1 à 5, seul(s) ou en combinaison avec d'autres médicaments ou substances actives pour la préparation d'un médicament destiné au traitement ou à la prophylaxie de troubles de la fonction intestinale.

13. Préparation pharmaceutique destinée à l'utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 5.

14. Préparation pharmaceutique destinée à l'utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 5, en combinaison avec une ou plusieurs autres substances actives pharmacologiques ou un ou plusieurs autres médicaments.
